# EUROPEAN PATENT SPECIFICATION

(11) **EP 0 454 735 B1**
(45) Date of publication and mention of the grant of the patent: **22.05.1996**
(21) Application number: 90902112.3
(22) Date of filing: 19.01.1990
(51) Int. Cl.: A61K 39/02, A61K 39/04, A61K 39/108, A61K 39/112, A61K 39/116, A61K 39/39

(54) **VACCINE COMPOSITION**
IMPFSTOFFZUSAMMENSETZUNG
COMPOSITION DE VACCIN

(30) Priority: 23.01.1989 AU 2368/89
(43) Date of publication of application: 06.11.1991
(73) Proprietor: AUSPHARM INTERNATIONAL LIMITED, Perth, W.A. 6000 (AU)
(72) Inventor: HUSBAND, Alan, James, New Lambton Heights, NSW 2305 (AU)
(74) Representative: Hildyard, Edward Martin
(86) International application number: AU9000014
(87) International publication number: WO9007935

(56) References cited:
- EP-A- 0 080 806
- EP-A- 0 242 205
- EP-A- 0 283 840
- WO-A-86/00019
- CA-A- 1 188 986
- GB-A- 1 401 588
- GB-A- 1 514 091
- US-A- 3 917 819
- US-A- 4 167 560
- VETERINARY IMMUNOLOGY & IMMUNOPATHOLOGY, vol. 5, no. 1, November 1983, Elsevier Science Publishers B.V., Amsterdam (NL); K. J. BEH et al., pp. 15-26/
- INFECTION & IMMUNITY, vol. 37, no. 1, July 1982, Washington, DC (US); D.F. KEREN et al., pp. 387-389/
- COMPTES RENDUS DE L'ACADEMIE DES SCIENCES, Serie D, vol. 280, 07 April 1975, Paris (FR); F. AUDIBERT et al., pp. 1629-1632/
- INFECTION & IMMUNITY, vol. 37, no. 3, September 1982; F.A. KLIPSTEIN et al., pp. 1086-1092/

## Description

This invention relates to a vaccine composition for stimulation of an effective IgA response in the intestine, in particular it relates to a vaccine for intraperitoneal use in commercial applications.

Attempts to potentiate production at mucosal surfaces of local antibodies against specified antigens have not been as successful as systemic immunization. This largely reflects lack of information regarding mechanisms of immune responses at local sites. Thus many systemic diseases are controlled by immunization of the systemic immune system yet the control of diseases by production of local antibodies at mucosal surfaces has not realised its full potential. In particular, diseases affecting young animals in intensive livestock production enterprises cause significant economic loss in these industries. For example in the pig industry occasional mortality reaching 30% can occur after weaning because of the lack of protection of animals at this age against environmental pathogens, usually E.coli.

The reasons for the high susceptibility at this stage are the lack of development of innate mucosal immunity in animals under one month of age and the commercial pressures for early weaning usually at four weeks of age. The sudden withdrawal of the blanket protection provided by maternal and milk antibodies, which in themselves have contributed to delayed onset of endogenous immunity in the neonates, leaves these animals highly susceptible to enteric infection. Despite numerous attempts to provide active protection by immunization the successes have been few. The reasons for this hinge on the lack of responsiveness of the intestine to environmental antigens reflected in poor responses to oral immunization. One method of immunization presently in use involves addition of E.coli antigens to the solid feed offered to young pigs during their suckling period. This long term exposure has resulted in protective immunity but is an extremely expensive feed additive vaccine and is usually sold with commercially prepared food products.

The present invention is directed to a novel vaccine composition which may be used, in one application, in the control of enteric disease in young animals.

Enteric disease remains one of the greatest causes of mortality and morbidity among domestic livestock, particularly young animals and especially in intensive rearing enterprises. The continual ingestion of potentially pathogenic material represents a barrage of antigens against which intestinal immune mechanisms provide the first line of defence. Effective protection of suckling neonates can be achieved by passive transfer of antibody via colostrum and milk after systemic immunization of the dam (Porter, 1979; Saif et al., 1983), however these animals remain at risk during the post-weaning period unless active intestinal immunity can be established.

In view of the demonstrated potential for the intestine to provide cellular and molecular effectors for remote mucosal sites (Husband, 1985) successful stimulation of IgA responses in the intestine can be expected not only to provide protection against enteric disease but, using appropriate antigens, also to protect remote sites. Attempts to achieve IgA responses in the intestine by oral immunization with non-replicating antigens have been characterized by ineffective responses of short duration, unless long term dosage is administered (Rowley, 1977; Newby and Stokes, 1984). Intraperitoneal (IP) immunization with antigen emulsified in Freund's complete adjuvant (FCA) on the other hand has produced effective intestinal IgA responses in rats (Pierce and Gowans, 1975), sheep (Beh et al., 1979; Husband, 1980; Husband et al., 1979) and pigs (Bennell and Husband, 1981; Husband and Seaman, 1979), presumably by producing inflammation of the intestinal serosa allowing antigen access to Peyer's patches. However FCA, which provides a mineral oil vehicle together with whole mycobacterial cell adjuvants, is unacceptable for routine use in commercial herds because it causes intense peritonitis and persistent mesenteric lesions when administered by the IP route. These lesions are unacceptable commercially in terms of carcass quality and are easily mistaken by meat inspection staff as indicative of infectious processes.

Audibert et al., 1975 disclose vaccines comprising peanut oil as a vehicle. Their effect is determined by measuring antibody titers in serum.

Enzootic pneumonia caused by Mycoplasma hyopneumoniae is a major health problem of pigs (Muirhead, 1987). In Australia, Pointon et al (1988) have shown that growth rate is reduced by 13% - 16% in pigs slaughtered at 85 kg, and these findings are similar to other reports from overseas (Goodwin 1963; Betts and Beveridge 1953; Muirhead 1987). Effective control of the disease requires recommended management procedures (Muirhead, 1987) and the use of antibiotics as a feed additive, fed either continuously or for intervals of about 1 - 2 weeks (Muirhead, 1987). However, the continuing use of antibiotics as a feed additive is under consideration (Webster, 1987) although the arguments against the use of antibiotics are not clear-cut (du Pont and Steele, 1987). An effective vaccine for controlling enzootic pneumonia would be of great benefit for the control of the disease. Numerous studies have assessed the immune response, lung pathology and microflora following immunization. Early observations indicated that older breeding animals had a lower prevalence of pneumonia, and that young animals with symptoms of pneumonia were free from pneumonia by the time they reached slaughter (Lannek and Bornfors, 1957). These findings suggested that immune mechanisms may be stimulated to offer long-term protection against further infection with M.hyopneumoniae. Several studies (Lannek and Bornfors, 1957; Goodwin et al, 1969) showed significant protection against challenge with M.hyopneumoniae in animals previously infected with M.hyopneumoniae. In one experiment this interval was extended to about 58 weeks (Goodwin et al, 1969) and still the animals showed a greater degree of protection than the control group. Further experiments investigated immunizing the sow and the role of colostral antibodies in protecting the offspring (Durisic et al, 1975; Kobisch et al, 1987) and these results, particularly of the latter study, look encouraging. A number of studies have also investigated the role of parenteral vaccination (Etheridge and Lloyd, 1982; Lam and Switzer, 1971; Goodwin and Whittlestone, 1973; Ross et al, 1983; Weng, 1985) and these results have also appeared encouraging. Despite this there is no effective vaccine available commercially.

The respiratory tract forms part of the common mucosal immune system (Bienenstock and Befus, 1980) and this is confirmed for the pig by further studies (Sheldrake et al 1988; Sheldrake, 1989 a, b). Those studies have indicated that it is possible to generate antigen-specific antibody producing cells in the tracheal lamina propria and specific antibody in trachael secretions after interoperitoneal (IP) immunization using Freund's Complete Adjuvant and subsequent tracheal challenge without adjuvant. In those studies it was propsoed that the IP immunization stimulated antibody precursor cells in the Peyer's patches. These cells entered the circulation, extravasated to all epithelial surfaces including the lamina propria of the trachea, and proliferated following further antigenic challenge of the trachea (Husband and Gowans, 1978). Recent results (Lloyd et al 1989) show that IP immunisation with a live attenuated strain of M.hyopneumoniae protect the lung against challenge with M.hyopneumoniae.

The present invention provides suitable vaccine vehicle preparations for IP use in domestic livestock to provide effective IgA immunity without undesirable side effects.

According to the present invention there is provided a vaccine composition for intraperitoneal administration to stimulate an IgA response, comprising an antigenically active substance in a vegetable oil vehicle, and optionally an adjuvant.

The present invention also provides a method of stimulating an IgA response in an animal which comprises intraperitoneal administration to said animal of a vaccine composition comprising an antigenically active substance in a vegetable oil vehicle, and optionally an adjuvant.

The antigenically active substance may for example, be selected to provide a killed E.coli antigen IP vaccine against postweaning enteritis in pigs, a Salmonella typhimurium antigen IP vaccine against postweaning enteritis in lambs, or a killed Mycoplasma hyopneumoniae IP vaccine against porcine enzootic pneumonia. It will be appreciated, however, that the antigenically active substance is not restricted to these particular examples and can in fact be selected from known antigens depending upon the nature of the immune response required.

In work leading to the invention, it has been demonstrated that a vehicle based on vegetable oil with natural emulsifiers (such as phosphatidyl choline) to achieve a stable oil-in-water emulsion can be administered intraperitoneally without causing any apparent mesenteric lesions or other undesirable side effects. To provide adjuvant activity in these formulations, either saponin, whole killed mycobacterium or purified mycobacterial cell wall extracts (muramyl dipeptide - MDP) may be used. These formulations have been shown in small animal experiments to be equivalent in promotion of IgA responses from the intestine to the previously used Freund's adjuvant formulations. Field trials have also indicated that using appropriate serotypes of E.coli in these formulations, substantial benefits in reduced mortality and morbidity in pigs due to post weaning enteritis can be achieved.

Although both saponin and MDP have been demonstrated to be effective adjuvants, MDP is considered to be superior in view of its ability to generate a response with a higher IgA component. Saponin may be of greater benefit when used in association with a membrane-bound antigen in view of its previously demonstrated ability to promote responses to sheep red blood cells but not bovine serum albumin, interpreted as a reflection of its binding affinity for membrane cholesterol (Bomford, 1980). It has also been shown to be a particularly good adjuvant for protozoal vaccines (Mitchell et al., 1979). Its routine use may be limited however by its reported haemolytic effects (Glauert et al., 1962) although there was no evidence of haemolysis in the present experiments. The ability of saponin to promote responses to membrane-presented antigens provided the rationale for its incorporation into liposome vesicles but this adjuvant/vehicle combination has not been found to be as effective as the vegetable oil emulsion in promoting IgA responses after IP administration.

The efficacy of MDP as an adjuvant for both IP and intra-duodenal (ID) presentation as found in the present invention is in accord with other reports (Kiyono et al., 1982). MDP is the purified peptidoglycan moiety of the cell wall of mycobacterium and performs equally well whether presented in its purified form or as a whole killed mycobacterium additive. Its incorporation into a vegetable oil emulsion has enabled its adjuvant effects to be expressed while held in the mesentery in depot form, but without eliciting the gross granulomatous lesions caused by the mineral oil component of FCA.

Preferably the vegetable oil used as the vehicle is safflower oil or sunflower oil, however it will be appreciated that this invention is not restricted to these particular oils. A particularly preferred vehicle/adjuvant combination for IP use in accordance with this invention is based on a vegetable oil emulsion with a purified muramyl dipeptide or killed M.bovis adjuvant.

The vaccine composition of this invention may be formulated as a stable vegetable oil emulsion, or in the form of liposomes.

The fact that IP administration of vaccines can now be considered as a practical alternative to oral immunization for stimulating IgA responses in the intestine of domestic livestock species, for example, using an MDP adjuvanted vegetable oil vaccine described here, has potential beyond its immediate benefit in control of enteric disease. There is now convincing evidence that both precursor cells of IgA specificity and molecular IgA originating from the intestine play an important role in protection of remote mucosal sites, particuarly the respiratory tract, urinary tract and in some species the mammary gland (Husband, 1985). Harnessing this potential depends on satisfactory stimulation of an intestinal response. Thus an IP vaccine incorporating appropriate relevant antigens could assist in control of diseases at many mucosal sites.

The following detailed description contained in the Examples demonstrates the effectiveness of vaccine compositions of this invention.

### EXAMPLE 1:

In this example, two adjuvants, saponin (McColm et al., 1982) and muramyl dipeptide (MDP) (Kiyono et al., 1982) were tested, chosen on the basis of their availability, ease of preparation and demonstrated adjuvanticity in systemic immunization applications. The various antigen-adjuvant combinations were injected either in vegetable oil emulsion or liposome (Shek and Sabiston, 1981) vehicles representing biodegradable alternatives to the mineral oil component used in FCA.

### A. MATERIALS AND METHODS:

### A.1 Animals

Adult inbred PVG strain rats were used to assess responses to the various vaccine combinations. In some experiments, selected preparations were administered to sheep and adult Border Leicester X Merino crossbred wethers were used.

### A.2 Vaccine Formulations

For all experiments the soluble protein antigen ovalbumin (OVA) (Grade V, Sigma) was used. The lyophilised protein was dissolved in phosphate buffered (pH 7.3) saline (PBS) and, with the exception of liposome preparations, the concentration was adjusted such that all vaccine formulations contained 500 µg OVA per IP dose for rat experiments and 50 mg per dose for sheep. For liposome preparations the aqueous phase contained 10 mg/ml OVA (see below).

The adjuvants tested were added to OVA solutions as follows: saponin was dissolved in dimethyl sulphoxide and added to aqueous phase OVA to provide a final concentration of 2 mg/ml saponin and 10% v/v dimethyl sulphoxide (McColm et al., 1982); MDP (N-acetylmuramyl-L-alanyl-D-isoglutamine, Sigma Chemical Co., St. Louis, USA) was added to OVA solution to a final concentration of 1 mg/ml (Kiyono et al., 1982). As an alternative source of MDP in some experiments heat killed Mycobacterium bovis (CSL, Melbourne, Australia) was added at the rate of 0.5 mg/ml.

Aqueous phase preparations were either emulsified in safflower oil or incorporated into liposome vehicles. Stable oil-in-water emulsions were prepared by addition of equal volumes of oil and adjuvanted protein solutions and the natural emulsifier phosphatidyl choline (obtained as soybean extract lecithin, Norganic-Anaheim, USA) added at 7.5% v/v. This mixture was emulsified by repeated syringeing through a 19 gauge needle. OVA-containing liposomes were prepared by the method of Shek and Sabiston (1981). Lipid phase, consisting of dipalmitoylphosphatidylcholine, cholesterol and phosphatidic acid in molar ratios of 2:1.5:0.2, was dried onto flasks using a rotary evaporator. Aqueous phase (containing OVA (10 mg/ml), MDP (1mg/ml) and saponin (2 mg/ml) in PBS) was entrapped in lipid vesicles by vigorous shaking for 5 min. after addition to the flask. Liposomes were washed twice in PBS by centrifugation at 1000 g for 5 min. and resuspended in an equal volume of PBS.

### A.3 Immunization Procedures

Immunization protocols were based on those previously established, using OVA+FCA, for the stimulation of IgA responses in rats (Pierce and Gowans, 1975) and sheep (Husband et al., 1979). Rats were primed IP by injection of 0.4 ml of vaccine into the peritoneal cavity and then challenged intraduodenally (ID) 14 days later by injection of 0.5 ml OVA (10 mg/ml), with or without adjuvants, directly into the lumen of the duodenum exposed by laparotomy. They were killed 5 days later and tissue collected. Previous studies have indicated that contrary to the situation in rats, sheep produce an IgA specific response to IP injection of OVA+FCA without the need for subsequent ID challenge (Husband et al., 1979). Thus sheep were immunized by a single IP injection of 10 ml vaccine and were sacrificed 14 days later. At post mortem all animals were inspected for mesenteric lesions.

### A.4 Enumeration of anti-ovalbumin-containing cells (AOCC)

AOCC and their class specificity were detected in sections of intestine (mid-jejunum) collected after sacrifice. Tissues were processed by cold ethanol fixation and paraffin embedding (Sainte-Marie, 1962). A double fluorochrome labelling technique was used to stain simultaneously for AOCC and IgA isotype specificity. All tissues were sequentially incubated with OVA (1 mg/ml) and fluorescein isothiocyanate (FITC)-conjugated rabbit anti-OVA. Rat tissues were then incubated with goat anti-rat IgA (heavy chain specific). The preparation of these reagents has been described previously (Husband and Dunkley, 1985; Sheldrake and Husband, 1985). Sections were washed extensively in PBS after each staining step. Cells were observed by narrow band selective excitation of FITC or TRITC using incident light illumination. AOCC were enumerated in scans of 1 field diameter width (330 µm at x500 magnification) from the epithelium to the serosa in 50 fields and results expressed as AOCC per cm of intestine (1 cell/scan = 30.3 cells/cm).

### B. RESULTS

All preparations were initially screened in rats for their ability after IP injection to prime for an IgA-specific intestinal AOCC response following ID challenge with OVA in PBS. The bench-mark for comparison was OVA in FCA, an adjuvant/vehicle combination which has been demonstrated previously to provide protective IgA immune responses in the intestine after IP administration (Pierce and Gowans, 1975; Husband, 1980; Husband and Seaman, 1979). The results in Table 1 confirm the efficacy of this vaccine in establishing an IgA-specific AOCC response (Experiment 1).

**TABLE 1**

| Intestinal AOCC response in rats following IP administration of OVA with various adjuvant/vehicle preparations. | | | | | |
|---|---|---|---|---|---|
| Expt. | IP | ID | AOCC/cm | % IgA AOCC | No. Rats |
| 1. | OVA+FCA | OVA | 102.3 ± 5.4 | 79.7 ± 2.1 | 13 |
| 2. | OVA | OVA | 4.1 ± 1.4 | - | 13 |
| 3. | OVA+OIL | OVA | 6.4 ± 3.6 | - | 9 |
| 4. | OVA+SAP | OVA | 28.7 ± 9.1 | 60.0 ± 5.4 | 9 |
| 5. | OVA+MDP | OVA | 43.3 ± 5.9 | 57.0 ± 14.5 | 4 |
| 6. | OVA+SAP+OIL | OVA | 53.6 ± 14.4 | 30.8 ± 9.54 | 4 |
| 7. | OVA+MDP+OIL | OVA | 80.2 ± 3.9 | 82.7 ± 1.59 | 12 |
| 8. | OVA+M.bovis+OIL | OVA | 77.5 ± 5.7 | 65.4 ± 2.6 | 4 |
| Values are means ± standard error of data from the number of rats indicated. (FCA - Freund's complete adjuvant; OIL = safflower oil; SAP = saponin; MDP = muramyl dipeptide). | | | | | |

On the other hand OVA given IP without either adjuvant or vehicle generated a very small response in the intestine (Experiment 2) and this was not improved if the OVA was delivered in a stable emulsion with vegetable oil vehicle (Experiment 3). The response was markedly improved however by addition of the adjuvants saponin or MDP to OVA (Experiments 4 and 5) and when these antigen/adjuvant mixtures were incorporated into a vegetable oil emulsion (Experiments 6 and 7) the response was further improved, although not to the level of that achieved with OVA+FCA. MDP was marginally superior to saponin in terms of the total AOCC response but the use of MDP adjuvant resulted in a higher proportion of AOCC of IgA specificity. The possibility of using whole killed M.bovis organisms as a substitute for MDP was investigated (Experiment 8), and this formulation delivered in vegetable oil emulsion produced an AOCC response equivalent to that obtained with OVA+MDP in vegetable oil emulsion but with a slightly reduced IgA component.

At post mortem examination rats given OVA+FCA (Experiment 1) had extensive mesenteric granulomatous lesions with multiple adhesions. However, none of the animals given any of the other vaccine formulations shown in Table 1 had any evidence of peritonitis or lesions and their mesenteries appeared normal in all respects.

The results in Table 1 show that both saponin and MDP have adjuvant activity for IgA responses to OVA when given IP, especially if delivered in an oil emulsion, but that MDP is marginally superior in this regard. Since MDP has also been used effectively as an oral adjuvant for IgA responses (Kiyono et al., 1982) further experiments were undertaken to determine whether the responses obtained with MDP or saponin delivered IP in oil emulsions could be improved if MDP was also added to the ID challenge. The data in Table 2 indicate that greatly enhanced AOCC responses were achieved by these treatments which exceeded those obtained in rats immunized IP with OVA+FCA and challenged with OVA in PBS (Experiment 1, Table 1). Again, the rats immunized IP with vaccine containing MDP responded marginally better than those given the saponin vaccine, although in this case the IgA component was reduced.

**TABLE 2**

| Effect of addition of MDP adjuvant to ID challenge on the intestinal AOCC response in rats. | | | | | |
|---|---|---|---|---|---|
| Expt. | IP | ID | AOCC/cm | %IgA AOCC. | No. Rats |
| 9. | OVA+SAP+OIL | OVA+MDP | 110.0 ± 10.5 | 86.9 ± 3.6 | 6 |
| 10. | OVA+MDP+OIL | OVA+MDP | 123.4 ± 8.9 | 65.6 ± 1.3 | 9 |
| Values are means ± standard error of data from the number of rats indicated. (OIL = safflower oil; SAP = saponin; MDP = muramyl dipeptide). | | | | | |

While these results show that MDP and saponin given in vegetable oil emulsion provide an alternative to FCA for IP stimulation of IgA responses, the success of liposomes in systemic antigen delivery (Kramp et al., 1979) suggested their potential as an additional alternative vehicle for IP use. Liposomes were prepared incorporating both MDP and saponin and injected IP (Table 3). However, after ID challenge with OVA with or without MDP, the resultant IgA-specific AOCC response in the intestine was only small (Experiments 11 and 12). A similar response occurred with OVA-liposomes were used for both the IP immunization and ID challenge (Experiment 13). No mesenteric lesions appeared in any of these animals at post mortem injection.

**TABLE 3**

| Effect of liposome vehicle for IP and/or ID delivery on intestinal AOCC response in rats. | | | | | |
|---|---|---|---|---|---|
| Expt. | IP | ID | AOCC/cm | %IgA AOCC | No. Rats |
| 11. | OVA/LIPOSOMES | OVA | 17.8 ± 5.7 | 36.7 ± 5.7 | 5 |
| 12. | OVA/LIPOSOMES | OVA+MDP | 22.4 ± 6.3 | 33.9 ± 4.6 | 4 |
| 13. | OVA/LIPOSOMES | OVA/LIPOSOMES | 18.4 ± 5.4 | 43.6 ± 3.2 | 5 |
| 14. | OVA+MDP+OIL | OVA/LIPOSOMES | 124.9 ± 13.9 | 79.3 ± 0.9 | 4 |
| Values are means ± standard error of data from the number of rats indicated. (OIL = safflower oil; SAP = saponin; MDP = muramyl dipeptide). | | | | | |

Liposomes did however appear to have a role in mucosal antigen delivery since rats immunized IP with OVA+MDP in vegetable oil but challenged ID with OVA-liposomes produced on outstanding response (Experiment 14), greater than that achieved by OVA+FCA and equivalent to that obtained by IP immunization with OVA+MDP in vegetable oil and ID challenge with OVA+MDP.

In view of the promising results obtained by incorporation of OVA+MDP in vegetable oil emulsions for IP administration, the extension of this to large animal use was investigated. In sheep, contrary to rats, a single IP dose of OVA+FCA is sufficient to stimulate a large population of AOCC in the intestine, about half of which are IgA-specific (Husband et al., 1979). Because of the prohibitive expense of scaling up to the dose required if commercial purified MDP were used, arid in view of the demonstrated capacity for killed M.bovis to replace MDP (Table 1) sheep were given an IP vaccine containing OVA emulsified in vegetable oil with M.bovis adjuvant. The data in Table 4 indicate that whereas OVA given IP without adjuvant or vehicle produced virtually no intestinal AOCC response in sheep, OVA+M.bovis in vegetable oil emulsion produced an AOCC response equivalent in magnitude to that observed with OVA+FCA, but with an elevated proportion of AOCC of the IgA isotype.

**TABLE 4**

| Intestinal AOCC response in sheep following IP administration of OVA with various adjuvant/vehicle preparations. | | | |
|---|---|---|---|
| IP | AOCC/cm | %IgA AOCC | No. sheep |
| OVA | 2.5 ± 2.5 | - | 4 |
| OVA+FCA | 87.0 ± 14.0 | 55.0 ± 3.5 | 6 |
| OVA+M.bovis+OIL | 68.9 ± 4.1 | 75.1 ± 3.7 | 4 |
| Values are means ± standard error of data from the number of sheep indicated. (FCA = Freund's complete adjuvant; OIL = safflower oil). | | | |

### C. DISCUSSION:

These experiments demonstrate that a vaccine formulation based on vegetable oil vehicle adjuvanted with whole M.bovis cells can be used to replace FCA in large animal applications. Thus an IP vaccine incorporating appropriate relevant antigens could assist in control of enteric diseases and, by virtue of the common mucosol immune system, contribute to immunity at other mucosal sites (e.g. lungs, urogenital tract, etc.).

### EXAMPLE 2:

In view of the success of the vegetable oil based formulation in stimulating AOCC responses in the intestine of both rats and sheep, experiments have been undertaken in pigs with a view to (a) demonstrating the potential of the vaccine formulation to produce IgA AOCC in the gut (Example 2) and (b) the ability of this formulation to stimulate a protective immune response against the organisms responsible for post-weaning enteritis (predominantly serotypes of E. coli) after administration to pigs prior to weaning (Example 3).

### A. MATERIALS AND METHODS:

### A.1 Animals

Landrace X Large White gilts aged 19-20 weeks were obtained from a commercial breeding herd.

### A.2 Vaccine Formulation

A vaccine containing OVA in vegetable oil vehicle with M.bovis adjuvant was prepared as described for sheep experiments (Section A.2 in Example 1). OVA+FCA was also prepared as described in Example 1.

Since previous experiments in pigs had established that oral challenge with soluble OVA containing DEAE-Dextran enhanced the IgA AOCC response after immunization with OVA+FCA (Bennell and Husband, 1981), additional experiments were undertaken to investigate the role of oral challenge in responses obtained with the vegetable oil alternative vaccine.

### A.3 Immunization Procedures

Animals were randomly allocated to one of four treatment groups as outlined in Table 5.

**TABLE 5**

| Experimental design for assessment of alternative vaccine formulations in pigs | | | | |
|---|---|---|---|---|
| Treatment Group | Day 0 | Day 14 | Days 15-20 | Day 21 |
| Group 1 | OVA + FCA | OVA | - | Kill |
| | IP | oral | | |
| Group 2 | OVA + FCA | OVA + Dex | OVA + Dex | Kill |
| | IP | oral | oral daily | |
| Group 3 | OVA + VEG | OVA | - | Kill |
| | IP | oral | | |
| Group 4 | OVA + VEG | OVA + Dex | OVA + Dex | Kill |
| | IP | oral | oral daily | |
| Notes: n = 5 per group OVA + FCA = 1.5 ml OVA (10 mg/ml) + 1.5 ml Freund's complete adjuvant. OVA + VEG = 1.5 ml OVA (10 mg/ml) + 1.5 ml vegetable oiul adjuvant. OVA oral = 50 ml OVA (10 mg/ml) by stomach tube OVA oral + Dex = 50 ml OVA (10 mg/ml) + 2.5 g DEAE-Dextran by stomach tube. | | | | |

At sacrifice animals were bled and intestinal tissues (mid-jejunum) obtained for histological assessment of AOCC responses as described in Example 1. Sections were stained by sequential incubation with OVA (1mg/ml), fluorescein isothiocyanate (FITC)-conjugated rabbit anti-OVA and tetramethylrhodamine isothiocyanate (TRITC)-conjugated rabbit anti-porcine IgA (heavy chain specific). In addition, antibody titres to OVA in intestinal secretions and blood plasma were assayed using an isotype-specific anti-OVA ELISA.

### B. RESULTS

The AOCC response following vaccination in each of the groups is shown in Table 6.

As described previously the IP administration of OVA+FCA followed by oral challenge resulted in a substantial IgA specific AOCC response in the intestine of pigs and the incorporation of DEAE-Dextran in the oral challenge enhanced the number of AOCC and the proportion which were IgA specific. Pigs receiving vegetable oil vaccine also produced an AOCC response which was not as great as in pigs receiving OVA+FCA but had an equivalent IgA component.

As expected, all pigs receiving FCA vaccine developed lesions and adhesions in the peritoneal cavity. However pigs receiving the vegetable oil vaccine had no lesions and no abnormalities were detected at post mortem examination.

The IgA-specific and IgG-specific anti-OVA titres in blood and intestinal secretions are shown in Tables 7 and 8 respectively.

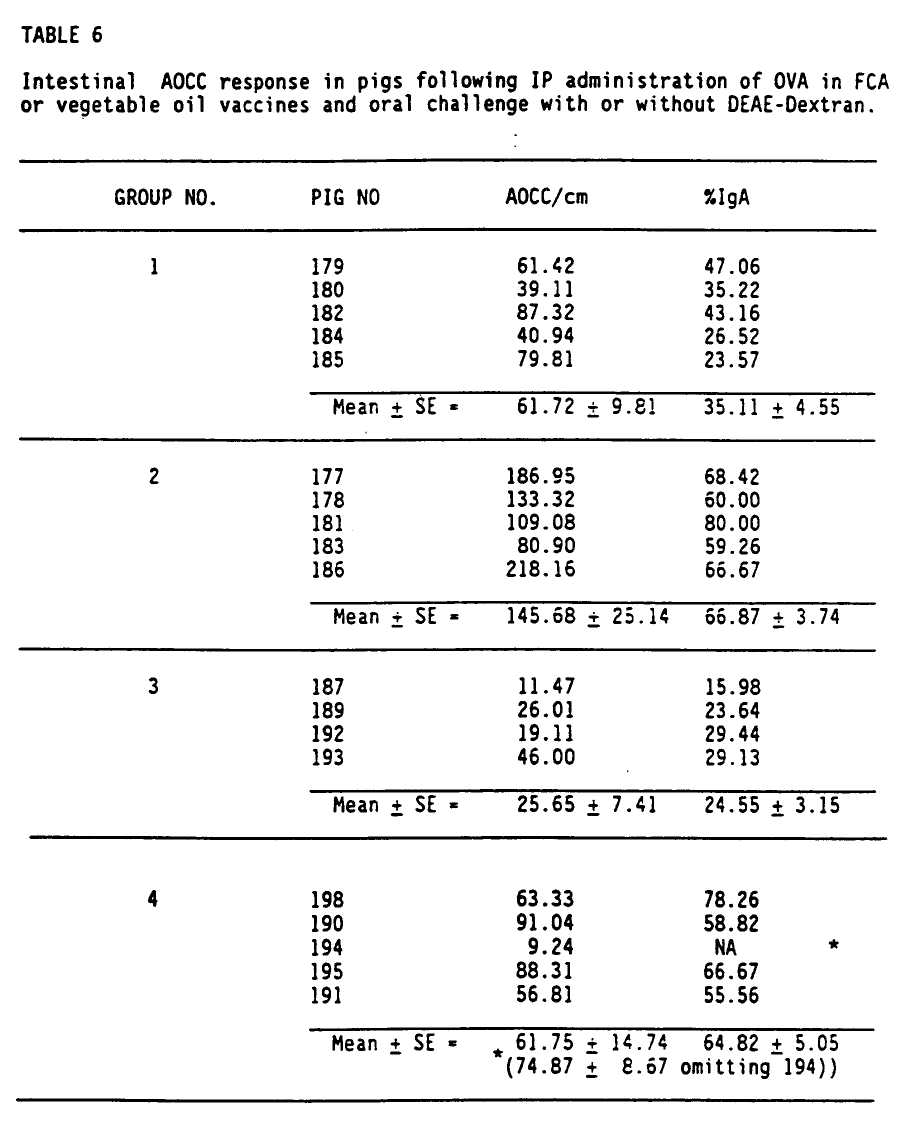

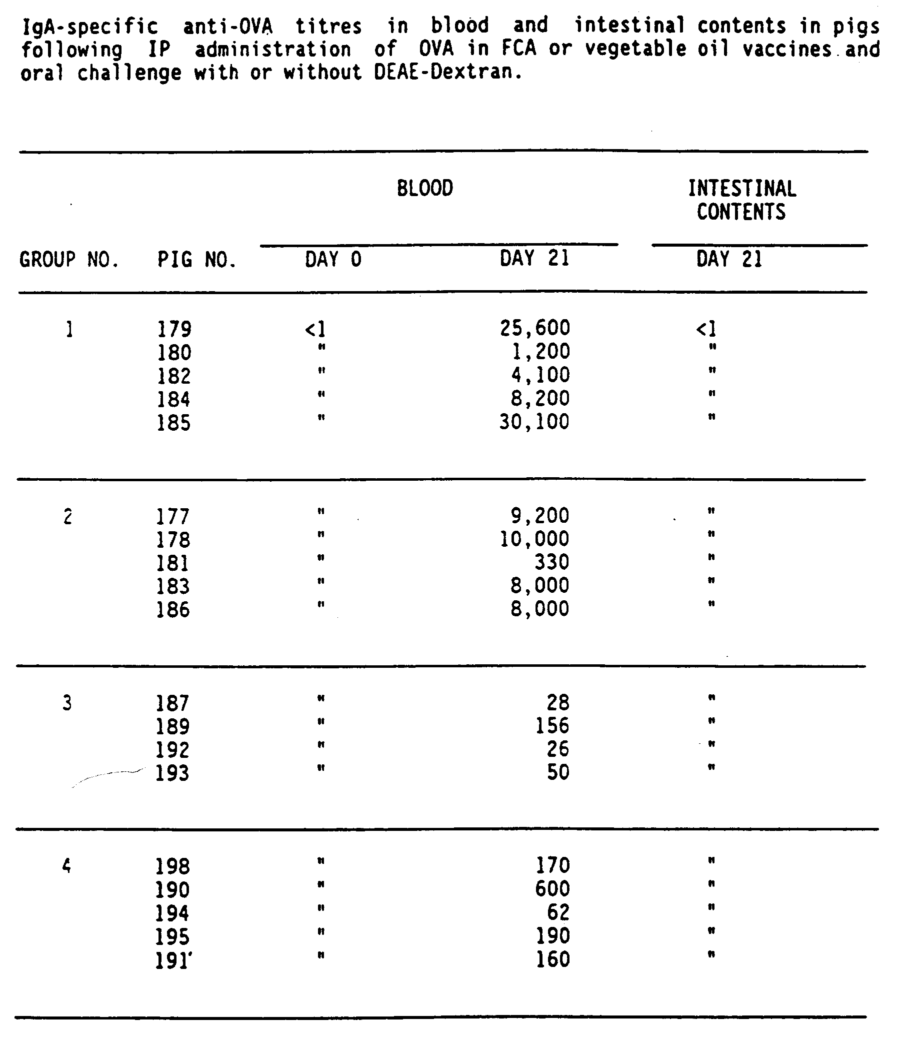

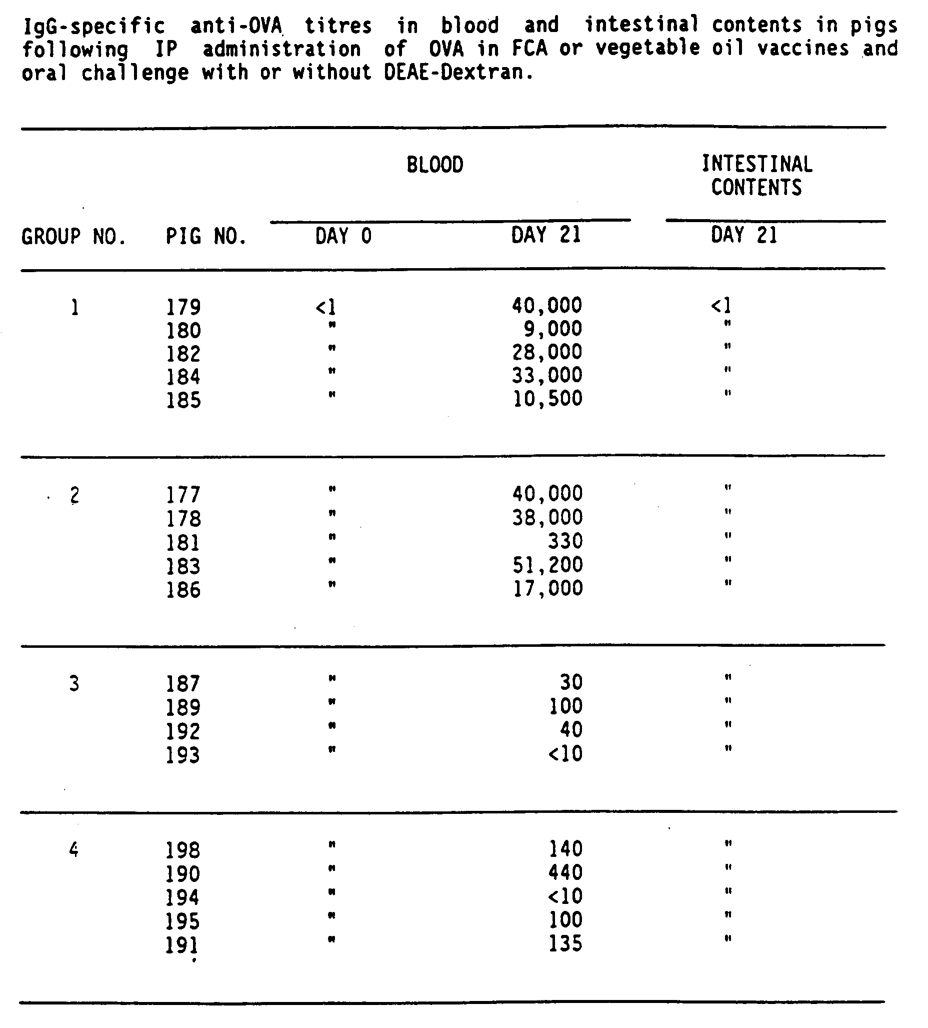

### C. DISCUSSION

Consistent with previous observations (Bennell and Husband, 1981), IP administration of OVA+FCA primed the gut of pigs such that a substantial AOCC response occurred following oral challenge. However only 35% of cells were IgA specific. Incorporation of DEAE-Dextran in the oral challenge enhanced the AOCC response and increased the proportion of cells producing IgA-specific antibody.

OVA administered in M.bovis-adjuvanted vegetable oil vaccine stimulated a smaller response when oral challenge was given as OVA alone but the response was substantially increased by repeated oral challenges containing DEAE-Dextran.

The reason for the enhanced IgA-specific AOCC response with DEAE-Dextran is unclear. DEAE-Dextran is a polycation soluble adjuvant and was used by Beh at al (1979) to enhance the intestinal response of sheep to intraintestinal antigen infusion. It is possible that the presence of dextran encourages precocious differentiation of immature B cells resulting in their switch to IgA production at an earlier time. Although oral MDP was an effective immunostimulant in the preceding experiments its use in large animals is prohibited by cost factors. DEAE-dextran may therefore be an acceptable alternative if an oral adjuvant is required. However for practical applications where bacterial rather than soluble protein antigens are used, oral adjuvants may not be necessary and would be unlikely to gain industry acceptance in intensive production enterprises.

The vegetable oil vaccine failed to stimulate an anti-OVA antibody response in serum of the magnitude observed with OVA+FCA. However since the vaccine is targeted at producing a local response in the intestine the circulating levels of antibody are largely irrelevant.

The most appropriate site to measure antibody responses would be in intestinal secretions since, based on results in Table 6, an IgA-specific AOCC response was produced in the intestinal lamina propria following vegetable oil vaccine and these antibodies are predominantly secreted into the intestinal lumen rather than serum. However no antibody responses were detected in intestinal contents from any animals in this experiment, probably because of the high rate of dilution of intestinal secretions with food and other digestive fluids.

However, on the histological evidence these data confirm that an M.bovis adjuvanted vegetable oil vaccine administered by IP injection can produce an effective IgA-specific anti-OVA response in the intestine of pigs, provided appropriate oral challenge occurs, and that this is achieved without peritoneal lesions or side effects.

### EXAMPLE 3 :

5 serotypes of E.coli expressing selected O and K antigens (0141, 0149, 0157, 08(67) and K81) were chosen on the basis of their incidence in field cases of the postweaning enteritis syndrome and incorporated into a vegetable oil vehicle. The resulting vaccine ("pentovax"*) has been administered IP to suckling pigs in commercial piggeries to assess its ability to stimulate immunity in the postweaning period against E.coli enteritis. Similar studies conducted under commercial conditions with a vaccine based on FCA had demonstrated potential for an IP vaccine to influence growth rates recorded later in the growth curve (Husband and Seaman, 1979). In addition to observing effects during the acute postweaning period, it was also important to follow production parameters during the entire growing period.
*Trade Mark

Although oral dosing has been shown to enhance the response of IP vaccinated pigs to OVA, it was considered impractical to incorporate oral vaccination steps into routine management systems in commercial piggeries, and, in any case, if enteric pathogens were endemic in a piggery environment a continuous oral challenge would occur naturally. Thus in the field trial presented here only IP immunizations were given.

### A. MATERIALS AND METHODS:

### A.1 Vaccine Preparation

### a. Bacteria

1. Inoculate nutrient broth tubes with bacteria from agar stock slopes of the appropriate serotype and incubate overnight at 37°C. Verify the purity of the culture by gram stain.
2. Subculture broth cultures onto blood agar plates and incubate for a further 18-24 hours at 37°C. Verify the purity of the culture again by gram stain.
3. Harvest bacteria into formalized saline (0.3% v/v paraformaldehyde in phosphate buffered (pH 7.3) saline) and incubate overnight at 4°C. At the end of this period inoculate the bacterial suspension onto blood agar plates to ensure sterility.
4. Adjust bacterial suspension photometrically to 10¹² organisms per ml and then precipitate by addition of 3.0 ml aluminium potassium sulphate (10.0% w/v) and 1.5 ml potassium hydroxide (7.4% w/v) per 100 ml of bacterial suspension. Allow the suspension to stand for 1 hr at room temperature then store at 4°C prior to use.

### b. Vaccine

1. Combine equal volumes of precipitated bacterial suspension and safflower oil.
2. Add heat killed Mycobacterium bovis at the rate of 0.25 mg/ml of oil/bacteria mixture.
3. Add phosphatidyl choline (lecithin) at the rate of 10.0% v/v.
4. Emulsify by repeated syringing or high speed blending until a stable viscous emulsion is achieved.
5. Store in sealed containers at 4°C until use. Shake well before and during use.

### A.2 Protocol

The general protocol is outlined in Table 9. Most piggeries use in-feed antibiotics as growth promoters and also to reduce the incidence of diseases such as postweaning enteritis.

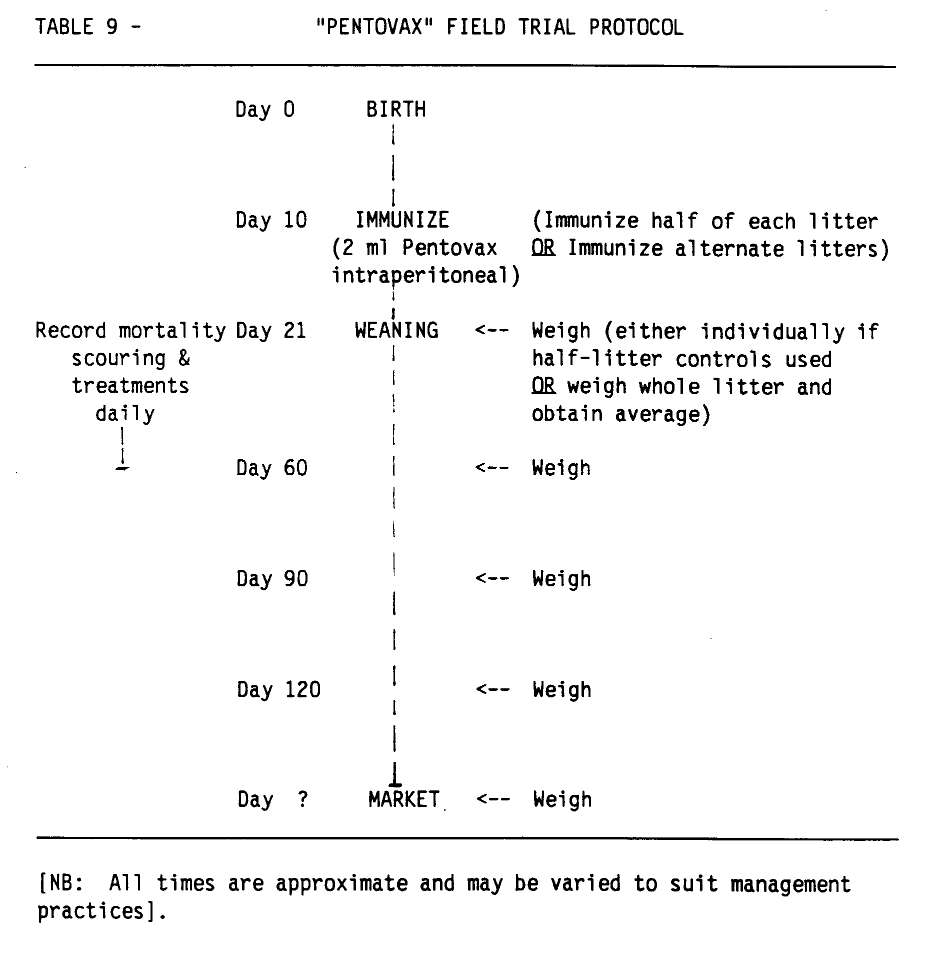

### B. RESULTS AND DISCUSSION

A preliminary trial was established primarily to establish the safety of the vaccine under commercial conditions but also to assess protection in an acute situation covering 10 days post weaning.

A total of 961 pigs were included in the trial and were allocated to vaccinated or control groups on a whole litter basis. The vaccinated group contained 439 pigs and the control group contained 522 pigs. The pigs were derived from 4 different breeding units. All pigs in this trial received a variety of in-feed antibiotics during the trial, Units 1, 2 and 3 receiving Bayo 100/Kitamycin 100/Auromycin 400 in feed and Unit 4 receiving Lincomycin in feed. Mean data is shown in Table 10.

All units except Unit 3 showed either improved results or no effect. The failure in Unit 3 may have been due to unusual disease burden or incorrect vaccine administration although these possibilities could not be confirmed. In any case mean data are calculated with and without Unit 3 data (Table 10). The low overall mortality figures indicate that during the period of this trial postweaning enteritis was not a problem in these units. The low mortality also establishes that the vaccine is safe and verbal reporting indicated no adverse symptoms in pigs after vaccination.

The low incidence of disease could have been attributable in part to the use of in-feed antibiotics but managment preference precluded their withdrawal in this trial. In any case it is anticipated that mortality and morbidity in the immediate postweaning period are not the only parameters which may benefit from vaccination and weight gain data over the entire growing period may have been advantaged but this was not assessed in this trial.

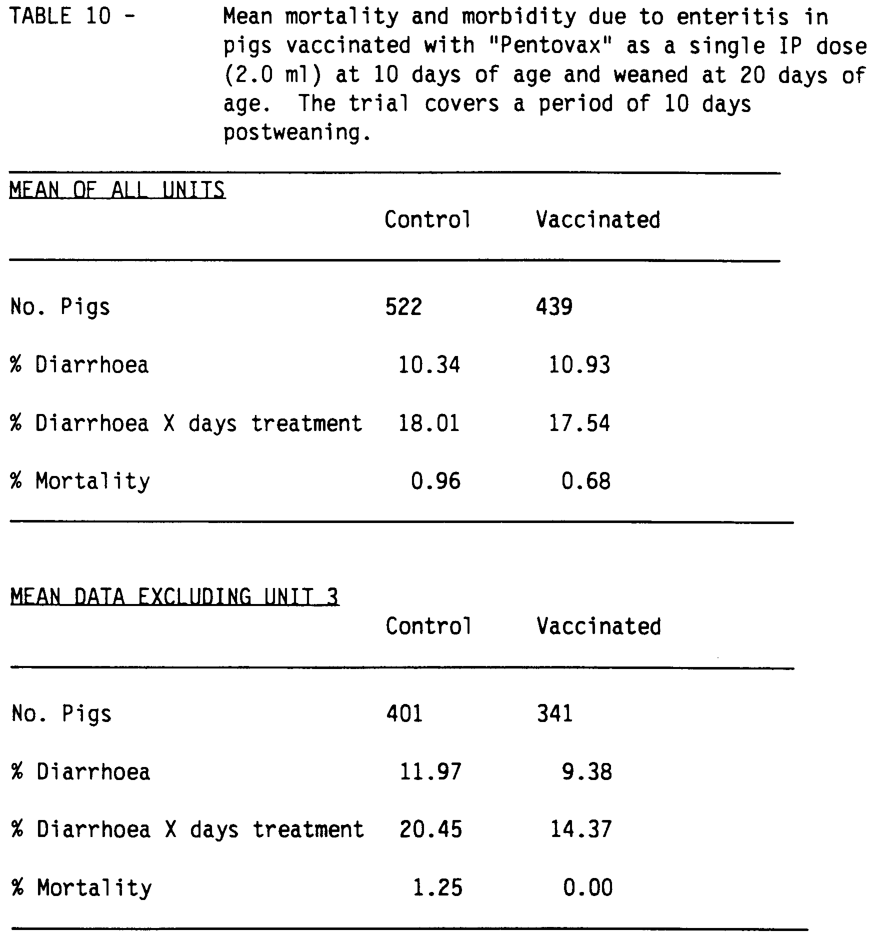

An additional trial was established incorporating medicated and unmedicated feed groups. This trial was conducted in two breeding units comprising 329 and 344 pigs respectively. The results are presented in Tables 11 and 12.

The features of this data are as follows:
1. As with all previous field trials there is an extremely low incidence of enteritis in any of the groups (only 4 deaths due to gastroenteritis out of a total of 673 pigs). Consequently there is very little effect of vaccine on mortality or morbidity under these conditions.
2. Considering the medicated feed groups only, there was no advantage in the vaccinated groups with respect to weight gain over the first 8 weeks postweaning but it should be emphasised that a weight gain advantage would not be expected to be reflected in this period but, on the basis of data obtained from a previous trial with a vaccine based on FCA adjuvant (Husband & Seaman 1979), this would be expected to occur as a catch-up effect when pigs are weighed later in the growing period.
3. There was a difference among the Plain Feed groups in % Total Weight Increase (ie. total weight of each group expressed as percentage of total weight at weaning, a figure which reflects weight gain and mortality effects) between vaccinated and control. This was most pronounced in Unit 2 at the 8 week period. If this were scaled up to the normal monthly output of around 1,000 pigs it would be reflected in a one tonne increase in live weight which would be of significant economic benefit. These data indicate that the sub-clinical E.coli problem is only being controlled by the use of in-feed antibiotics.
4. No adverse side effects were reported in any vaccinated pigs.

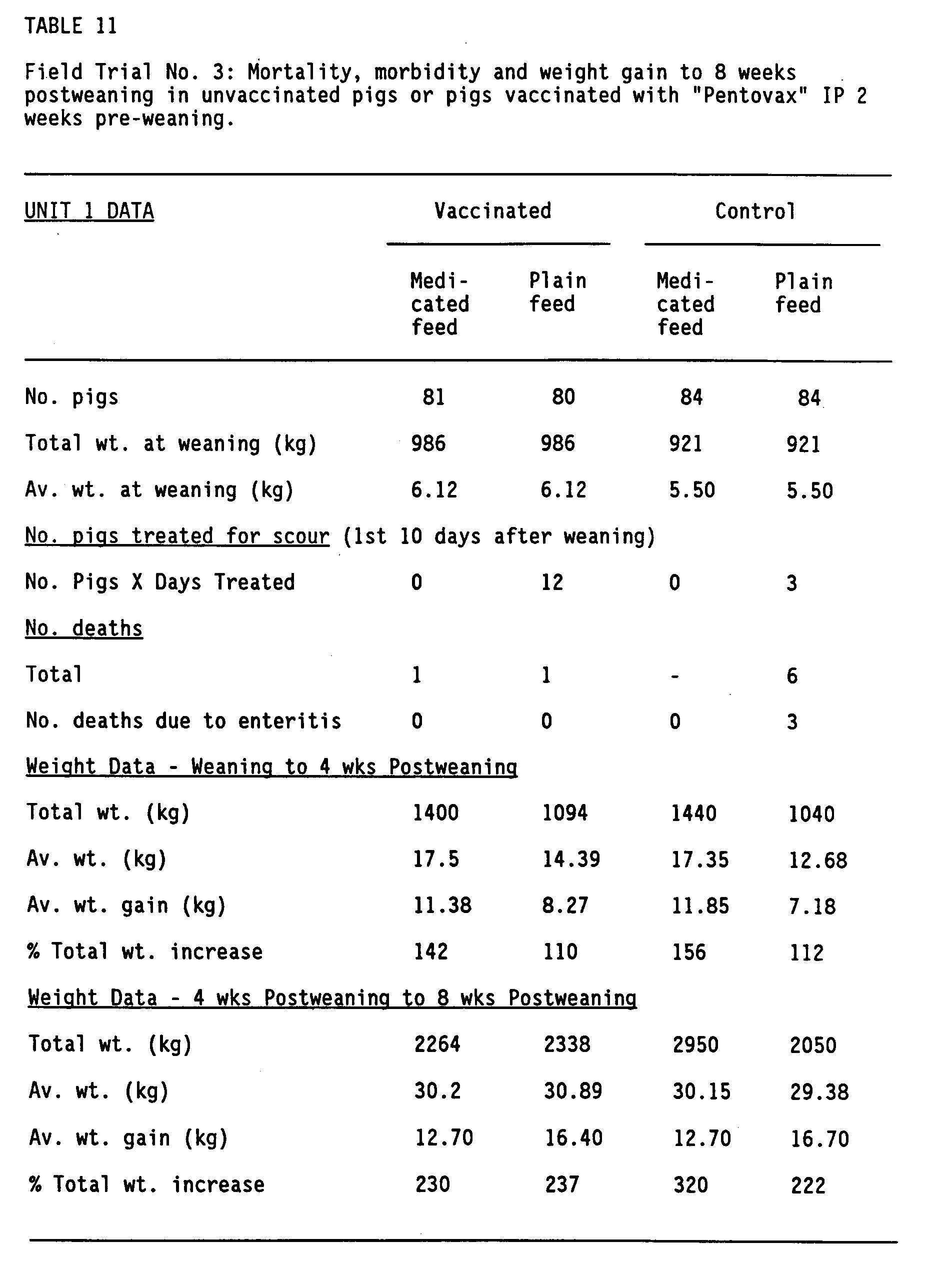

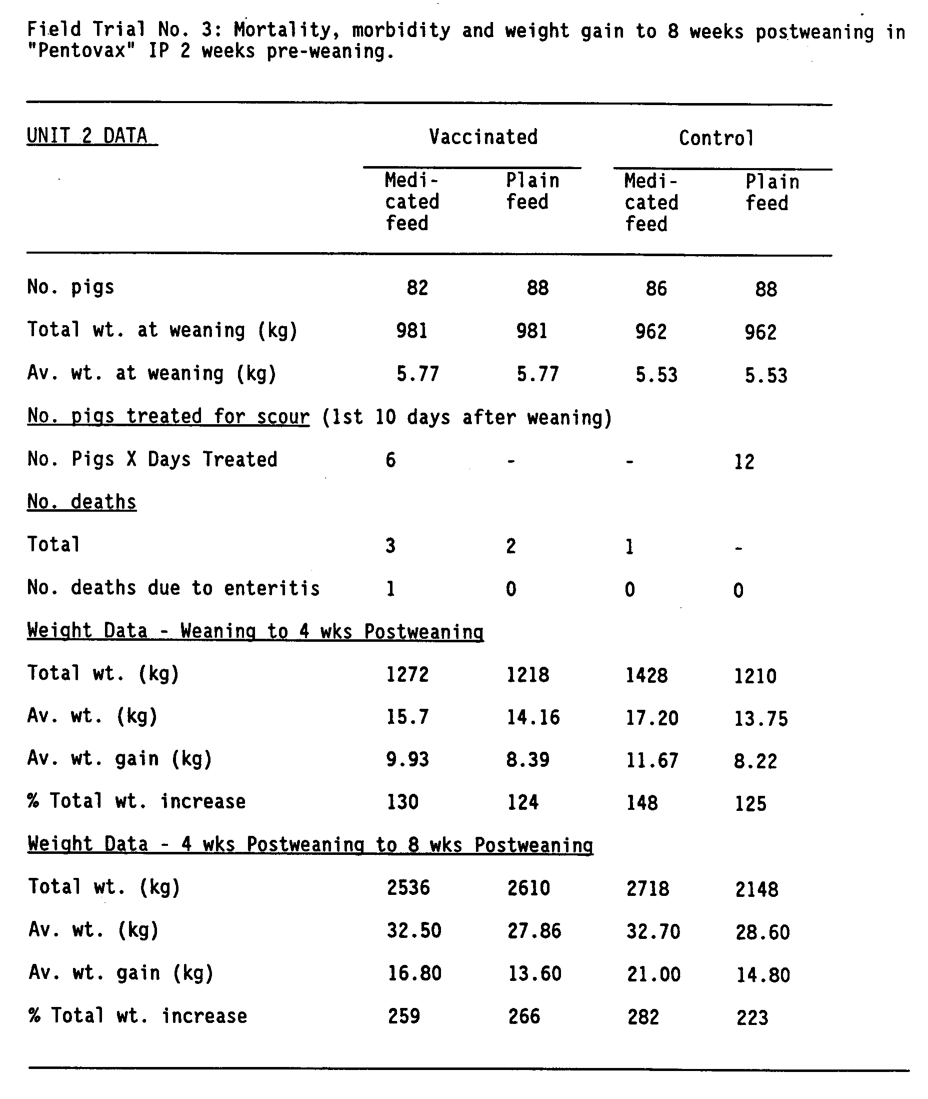

### EXAMPLE 4:

The following examples demonstrate that the IP method of vaccination using the M.bovis adjuvanted vegetable oil based vaccine will protect pigs in an environment where they are continually challenged by Mycoplasma hyopneumoniae.

### A. MATERIALS AND METHODS:

Study Herd: Investigations were undertaken in a 1950 sow intensive piggery located in central-western NSW. The herd consisted of four units of approximately 500 crossbred sows and their progeny. Commercially pelleted diets were fed ad libitum. Diets, stocking densities, pen sizes and shed ventilation have been previously published (Sheldrake et al, 1989). The herd was selected because it had a recurrent problem with pneumonia. During 1987/88, about 50% of all post-weaning deaths were attributed to pneumonia. Abattoir monitoring over the same period showed that 62% of pigs had pneumonic lesions with an average affected lung volume of 4.5%.
Cohort Selection and Management: Sixty six pigs from nine 4 week-old litters in one unit were weaned, weighted, ear tagged and randomly allocated on a litter and weight basis to three equally-sized groups. Pigs were excluded from the study if they weighed less than 6 kg or were suffering clinical illnesses such as arthritis. Group 1 (Medicated) was fed a medicated ration whereas Groups 2 and 3 (Vaccinated and Control respectively) were provided a non-medicated diet. Pigs were not given antibiotics either therapeutically or prophylactically other than those shown for Group 1. Physical segregation was maintained between Group 1 and the remaining two groups (which were randomly allocated into two pens) throughout the study, although pigs had through fence contact, and as well through fence contact with adjoining groups of pigs in the piggery. Surviving pigs were slaughtered at 163 days of age.
Data Collection: Monitoring commenced at weaning when pigs were weighed and blood sampled, and evaluated for clinical illnesses by the same veterinarian. These measurements were repeated every 4 weeks until slaughter. Final assessments were made 4 days prior to slaughter and the last blood sample was collected at slaughter. Carcass weights were determined after slaughter.
Vaccine Preparation: M.hyopenumoniae (J strain) was cultured in broth (Friis, 1977) by rotating continuously at 37°C until the change in pH resulted in the medium being honey coloured. The broth cultured was centrifuged at 10,000 g for 20 min. The pellet was subsequently washed 3 times in Tris buffered saline (TBS) (pH 7.4) in a similar manner.

The pellet was brought to a volume of 30 ml and formalin added to give a final concentration of 2%. The sample was stored overnight at 4°C and washed 3 times in TBS as described above. The wet weight of the pellet was determined and a concentration of 0.00866 g/ml TBS resulted. A 60 ml volume of this material was then emulsified with an equal volume of vegetable oil and M.bovis as described in Section A.1(6).
Vaccination Procedure: Twenty two pigs (Group 2) were vaccinated intraperitoneally at weaning (4 weeks) with a volume of 5 ml of the emulsified vaccine. These animals were vaccinated again 4 weeks later.
Sample Collection and Storage: Blood samples (5 - 10 ml) were collected from the anterior venacava or jugular vein into heparanized containers. Plasma was obtained and stored at -20°C until required for assaying.

### Enzyme-Linked Immunosorbent Assay (ELISA):

Antibody to M.hyopneumoniae in plasma was determined using an enzyme-linked immunosorbent assay (ELISA). Briefly, 96 well microtitre plates (NUNC, Copenhagen, Denmark) were incubated overnight at room temperature containing 100 µl of a purified fraction obtained from M.hyopneumoniae (Strain J). Subsequently, they were washed 8 times in Phosphate buffered Saline (PBS pH 7.2) containing 0.05% Tween *20 (ICI, Australia). A volume of 100 µl of 10% goat serum in PBS/Tween 20 was applied to the wells for 1 hr at room temperature and the plates washed as described above. Test sera were diluted twentyfold in PBS-Tween 20 containing 10% goat serum, and a volume of 100 µl applied in duplicate wells. Plates were allowed to incubate for two hours at room temperature before washing as described above. Shepp anti-pig (IgG) conjugated to Horse radish peroxidase (HRP) (Silenus Laboratories, Melbourne) was applied at a dilution of 1/500 in PBS Tween 20 with 10% goat serum and incubated for 1 hr at room temperature. After further washing the substrate 2,2'Azino Bis (3 Ethylbenzthiazoline-6-Sulphonic acid) Diammonium salt (ABTS) (Sigma, St Louis, MO.) was applied. The plates were read at 405 nm, after 10 min of development at room temperature using a Titretek Plate Reader (Flow Laboratories, Scotland). In the ELISA, all plates had four wells in which the test serum was replaced with a standard negative serum, obtained from pneumonia free pigs, and a further four wells with a positive serum, obtaining from herds known to be endemically infected.
Immunofluorescent Staining Procedures: Immediately after slaughter 0.5 cm cubes of tissue were collected from the lung areas adjacent to pneumonic lesions. The tissue was placed in 100% ethanol at 4°C, and processed through xylene and embedded in wax according to the method of Sainte-Marie (1962). Sections were cut to a thickness of 4µ. amd were processed through xylene and alcohol (Sainte-Marie, 1962). To determine the presence of M.hyopneumoniae or M.hyorhinis, rabbit antisera directed against these organisms was applied to separate sections, and allowed to incubate in a humid environment for 1 hr at room temperature. The sections were then washed in PBS and incubated with goat anti-rabbit fluoroscein isothiocyanurate (FITC) conjugated (Miles Laboratories, Melbourne), under similar conditions. The sections were further washed in PBS for an hour before examination for immunofluorescence.
Clinical Assessments and Pneumonic lesions: To quantify the amount of coughing, pigs were mixed and forced to exercise for 30 seconds. The during of coughing was recorded for each pig over the following 3 minutes. Pigs that died during the field study were necropsied and a likely cause of death established.
*Trade Mark

Pneumonic lesions in slaughtered pigs or in pigs dying during the study were recorded diagramatically and scores calculated according to the method of Goodwin et al (1969). The incidence of pleurisy and pericarditis at slaughter was recorded. Samples were collected from lesions where the aetiologicial agent was supected to be bacterial.

### B. RESULTS

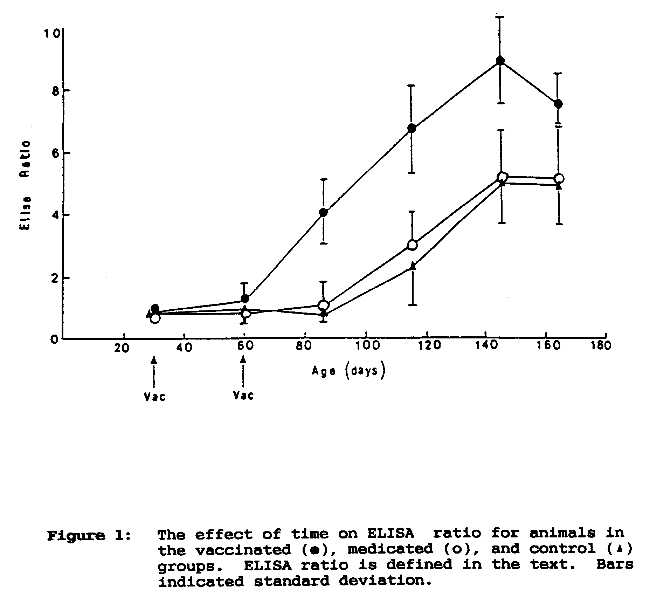

Figure 1 shows the M.hyopneumoniae antibody response in the three experimental groups. In vaccinated animals there was a significant (P<0.001) increase in the mean ELISA ratio between days 30 and 60, and this trend continued to day 144 of life. For the non-vaccinated medicated group and control group the ELISA ratio remained constant until day 115 of life when for both groups the ratio increased significantly (P<0.001). It increased further by day 144 (P<0.001) but then remained constant.

At day 60 and for each sampling thereafter, the mean ELISA ratio for the vaccinated group was significantly greater (P<0.001) than for the non-vaccinated (medicated and control) groups. There was no significant difference between ELISA ratios for these two groups at any time.

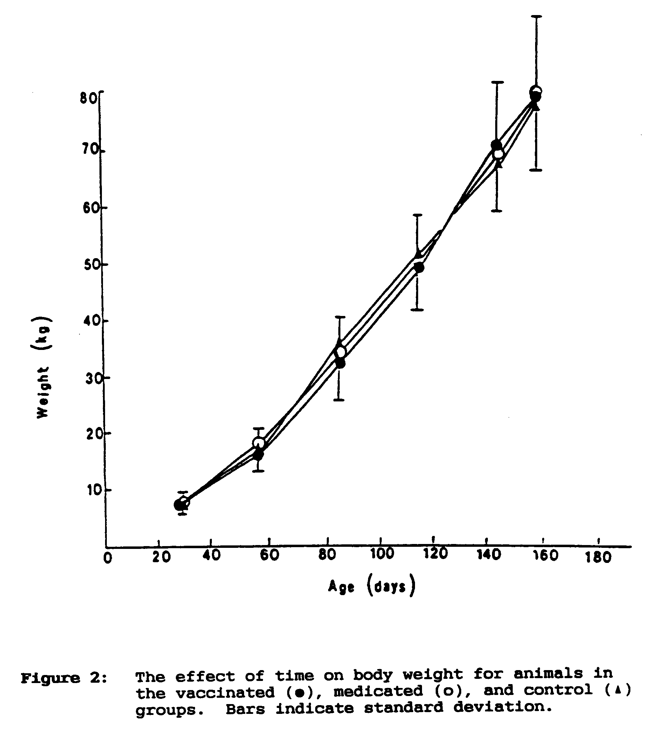

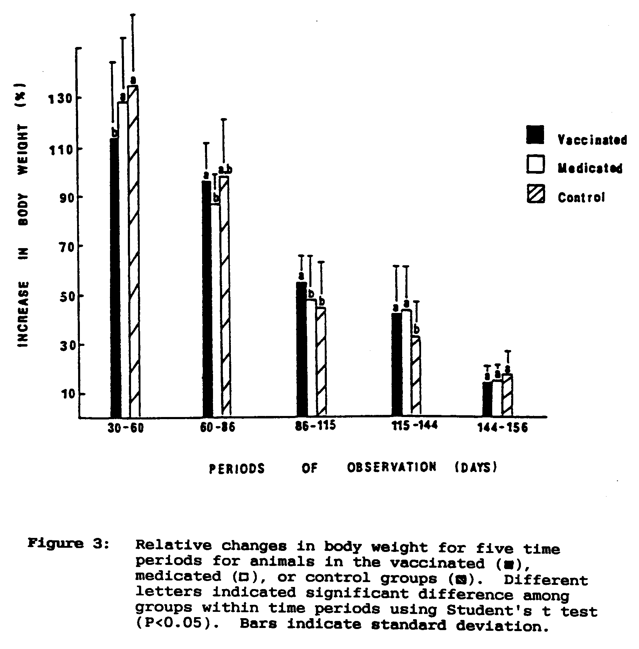

The body weights for animals in the three groups are presented in Figure 2. Because of the range in body weights within groups the standard deviation of the mean for each group is high and this masks relative differences among groups. In Figure 3 the weights are expressed as a percentage increase over the preceeding observation. They show that between days 30 and 60 the vaccinated group gained significantly less weight (P<0.01) than the medicated or control groups. However, between days 60 and 144 the vaccinated group gained significantly more weight (P<0.05) than the medicated or control group or both groups (days 86-115).

The descriptive statistics calculated for lung scores (Goodwin et al 1969) (Table 11) show that in the vaccinated group the mean lung score is significantly lower than the value of the medicated or control groups (P<0.05). The incidence of pleurisy and pericarditis and the number of lungs in which M.hyopneumoniae could be detected by immunofluorescence are shown in Table 12. All lungs were also assessed for M.hyorhinis by immunofluorescence, but no positive reactions were observed. Clinical assessments at regular intervals throughout the experimental period showed little difference among groups and there were no significant trends. Bacteriological examination of cultures from lungs indicated a similar distribution among groups, although two isolates of Actinobacillus (Haemophilus) pleuropneumonia (serovar 7) were detected in the vaccinated group and one in conjunction with Pasteurella multocida in the medicated group. There were 5 isolates of P.multocida in the control group, 3 in the medicated and one in the vaccinated group.

**TABLE 13**

| Range, Median and Mean ± SD of Goodwin Lung Scores determined at slaughter | | | | | |
|---|---|---|---|---|---|
| Group | Range | Median | Mean | SD | No. of Pigs |
| Vaccinated | 0-16.5 | 1.0 | 2.64a | 4.27 | 21 |
| Medicated | 0-55 | 3.5 | 9.36b | 14.00 | 22 |
| Control | 0-38 | 3.5 | 10.5b₁ | 12.40 | 21 |

| | | | | | |
|---|---|---|---|---|---|
| a-b significantly different using Student's t test, P<0.05 | | | | | |
| a-b₁ significantly different using Student's t test, P<0.01 | | | | | |

**TABLE 14**

| Number of animals with pleurisy or pericarditis at slaughter or with M.hyopneumoniae as judged by immunofluorescence. | | | | |
|---|---|---|---|---|
| | Pleurisy | Pericarditis | M.hyopneumoniae by IMF | No. of lungs examined. |
| Vaccinated | 5 | 5^{a} | 1^{d} | 21 |
| Medicated | 9 | 2^{a,b} | 5^{de} | 22 |
| Control | 5 | 0^{b} | 8*^{c} | 21 |
| Superscripts indicate significant difference in the distributions for pericarditis and presence of M.hyopneumoniae between vaccinated and control groups using X² analysis; a-b, (P<0.025); d-e, (P<0.005). | | | | |

| | | | | |
|---|---|---|---|---|
| * Only 20 lung sections were examined. | | | | |

### C. DISCUSSION

Following intraperitoneal vaccination at weaning and again 4 weeks later with formalin killed M.hyopneumoniae the ELISA ratio was elevated at day 60 and continued to rise until day 144. Animals in the vaccinated group had significantly lower pneumonic lung scores at slaughter (Table 11) and a greater proportion of animals with lung scores less than 5 or 20. It appears likely that the elevated levels of antibody present in serum reflected an increased level of antibody in respiratory tract secretion although this was not measured. Results from previous experiments (Sheldrake 1989 a, b) in which a similar immunization strategy was employed with ovalbumin as the antigen indicated that antibody levels in both serum and respiratory tract secretion (RTS) were elevated. This antibody was of both the IgG and IgA classes in RTS and serum. The anti-ovalbumin IgA in RTS was probably produced by a large population of antigen specific IgA plasma cells underlying the tracheal lamina propria induced by the IP immunization and tracheal challenge. It is likely that in this study both serum derived and locally derived anti-M.hyopneumoniae antibody found its way to the lung and trachea following IP immunization and the respiratory tract challenge provided by the colonizing M. hyopneumoniae.

The results show that lung pathology can be reduced following vaccination in a commercial herd under natural conditions of infection. This supports the early observations of Lannek and Bornfors (1957) of reduced lung pathology in animals previously showing symptoms of pneumonia. While the challenge experiments of Goodwin (Goodwin et al 1969, Goodwin and whittlestone 1973) indicated protection was possible when the antigen was prepared in adjuvant, in a later study Goodwin (1973) could find no evidence of protection under field conditions.

While the vaccine appears successful when judged by the degree of pneumonic lung score at slaughter, the definitive assessment of any field vaccine for pneumonia control must be slaughter weight. In this trial there were no significant differences among groups in body weights just prior to slaughter (Figure 2). This is clarified by Figure 3 which shows that in the period 30 to 60 days the vaccinated group gained significantly less weight than the medicated or control groups. It is possible that the 5 ml vaccine dose at 4 weeks of age inhibited the growth rate. During the second 30 day period following the second vaccination, this check in growth rate was not evident. In the third 30 day period the growth rate of the vaccinated group was significantly greater (P<0.05) than that of the medicated or control groups.

### EXAMPLE 5:

The aim of the present example was to repeat the work described in Example 4 but to try and overcome the growth depression which occurred after vaccination. To achieve this the vaccine volume was reduced to 2ml, although the antigen load remained the same. Pigs were vaccinated in a similar manner and at the same times. At the commencement of the trial there were 66 vaccinated pigs, 198 non-vaccinated pigs and 132 medicated pigs. The piggery was the same one as described in Example 4.

### A. RESULTS

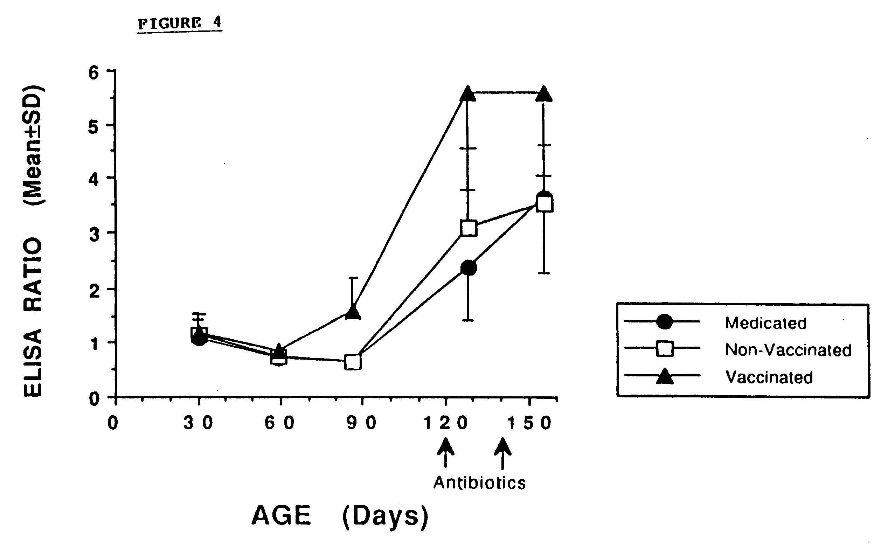

The results in Figure 4 show antibody titre results for the three groups. In the vaccinated group antibody levels were elevated after about two months.

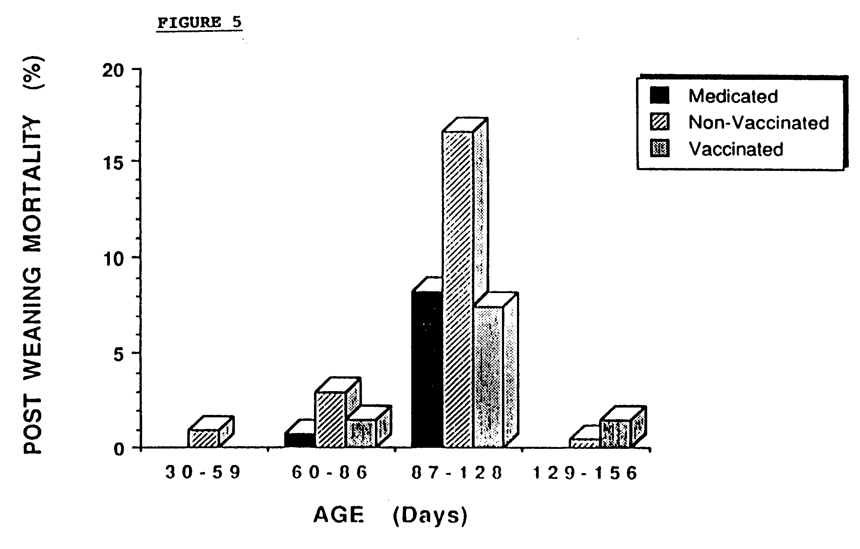

In Figure 5 is presented the weaning to slaughter mortality data. In the non-vaccinated group this represented a cumulative percentage of 21% which was significantly higher than the level of 10.6% for vaccinated or 9.1% for medicated pigs. For the last two groups there was no significant difference.

Table 13 presents the mean Goodwin Lung Score Data. Overall, there were no significant differences among the groups.

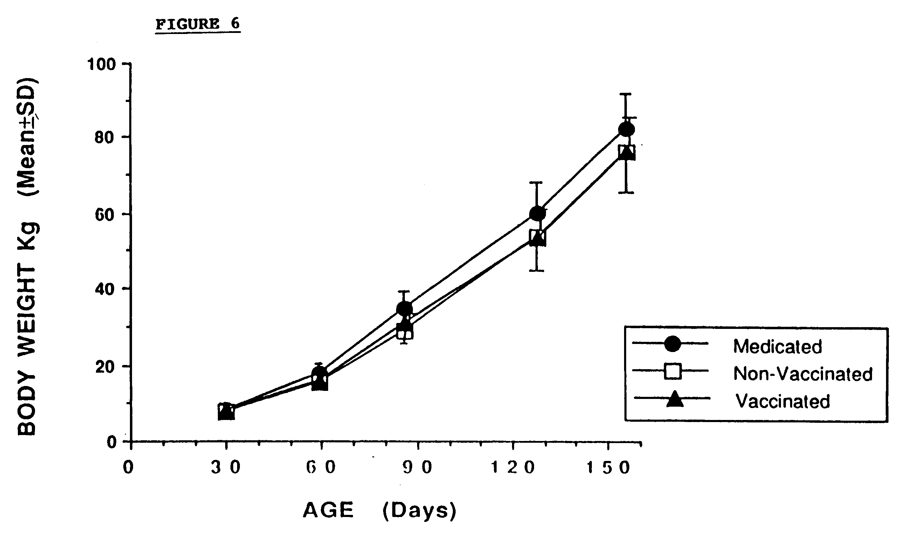

Figure 6 represents the mean body weight data for the three groups from weaning to slaughter. At slaughter the mean body weight of the medicated pigs was approximately 6kg greater than the vaccinated or non-vaccinated controls.

**TABLE 15**

| Mean + SD of Goodwin Lung Score and Pleuropneumonia Index (PI) | | | |
|---|---|---|---|
| Treatment | | Goodwin's Lung Score | PI |
| Medicated | Mean | 7.47 | 0.25 |
| | SD | 9.20 | 0.62 |
| | Number | 117 | 117 |
| Non Vaccinated | Mean | 7.80 | 0.30 |
| | SD | 10.20 | 0.67 |
| | Number | 154 | 154 |
| Vaccinated | Mean | 10.15 | 0.25 |
| | SD | 10.80 | 0.60 |
| | Number | 56 | 56 |
| There was no significant difference in mean lung scores or PI as judged by Student's t test. | | | |

### B. DISCUSSION

The results from this study highlight that vaccination is able to substantially influence M.hyopneumoniae antibody levels in serum. In this study however antibody levels increased to a level of only about a quarter of that observed in the previous study (Example 4) at 80 days. It is at this point and shortly after that pigs are becoming infected. Thus the lower levels of antibody in serum detected in this study probably accounts for the lower level of protection, as judged by Goodwin Lung scores compared with the results attained previously (Example 4). However, it should be noted that in the vaccinated group which received no medication, weaning to slaughter mortality was similar to that of the medicated non-vaccinated group. Moreover, while the vaccinated group weighed less at slaughter than the medicated group, no growth depression was observed following vaccination. Thus suggests that the smaller vaccine volume may have been less irritant, but on the other hand appears not to have maximized the antibody response.

It should be emphasized that in this piggery medication was extremely high, being 800g oxytetracycline/tonne in the finisher stage pigs. Thus, the reduction in mortality achieved by the vaccine when compared to this level of medication is quite considerable. Similarly with the growth curves. Thus it is likely that despite the lower slaughter weight in this herd vaccination may still be more cost-effective when the cost of medication is included.

### REFERENCES:

Beh, K.J., Husband, A.J. and Lascelles, A.K., 1979. Immunology, 37: 385-388.
Bennel, M.A. and Husband, A.J., 1981. Res. vet. Sci., 30: 353-356.
Betts, A.O. and Beveridge, W.I.B., 1953. Vet. Rec., 65: 515.
Bienenstock, J. and Befus, A.D., 1980. Immunol., 41: 249.
Bomford, R., 1980. Int. Archs Allergy appl. Immunol., 63: 170-177.
du Pont, H.L. and Steel, J.H., 1987. Rev. Infect. Dis., 9: 447.
Durisic, S., Maksimovic, A., Visacki, J., Knezevic, N. and Markovic, B., 1975. Acta. Vet. Yugoslavia, 25: 189.
Etheridge, J.R. and Lloyd, L.C., 1982. Res. Vet. Sci., 33: 188.
Friis, N.F., 1977. Acta. Vet. Scand., 18: 168.
Glauert, A.M., Dingle, J.T. and Lucy, J.A., 1962. Nature, 196: 953-955.
Goodwin, R.F.W., 1963. Br. Vet. J., 119: 298.
Goodwin, R.F.W., 1973. Br. Vet. J., 129: 465.
Goodwin, R.F.W., Hodgson, R.J., Whittlestone, P. and Woodhams, R.L., 1969. J. Hygiene (Camb), 67: 193.
Goodwin, R.F.W. and Whittlestone, P., 1973. Br. Vet. J., 129: 465.
Husband, A.J., 1980. Vet. Immunol. Immunopath., 1: 277-286.
Husband, A.J., 1985. Mucosal immune interactions in intestine, respiratory tract and mammary gland. In Progress in Veterinary Microbiology and Immunology. Ed. R. Pandey., S. Karger, Basel., Vol. 1, pp. 25-27.
Husband, A.J., Beh, K.H. and Lascelles, A.K., 1979. Immunology, 37: 597-601.
Husband, A.J. and Dunkley, M.L., 1985. Immunology, 54: 215-222.
Husband, A.J. and Gowans, A.J., 1978. J. Exp. Med., 148: 1146.
Husband, A.J. and Seaman, J.T., 1979. Aust. vet. J., 55: 435-436.
Kiyono, H., McGhee, J.R., Kearney, J.F. and Michalek, S.M., 1982. Scand. J. Immunol., 15: 329-339.
Kobisch, M., Quillien, L., Tillon, J.P. and Wroblewski, 1987. Ann. Inst. Pasteur Immunol., 138: 693.
Kramp, W.J., Six, H.R., Drake, S. and Kasel, J.A., 1979. Infect. Immun., 25: 771-773.
Lam, K.M. and Switzer, W.P., 1971. Am. J. Vet. Res., 32: 1737.
Lannek, N. and Bornfors, S., 1957. Nord. vet. Med., 9: 91.
Lloyd, L.C., Cottew, G.S. and Anderson, D.A., 1989. Aust. Vet. J., 66: 9-12.
McColm, A.A., Bomford, R and Dalton, L., 1982. Parasite Immunol., 4: 337-347.
Mitchell, G.H., Richards, W.H.G., Voller, A., Dietrich, F.M. and Dukor, P., 1979. Bull. W.H.O., 57 (Suppl. 1): 189-197.
Muirhead, M.R., 1987. Respiratory Diseases. In Proceedings of Pig Refresher Course No. 95. Post Graduate Foundation, Faculty of Veterinary Science, Sydney, Australia, p. 561.
Newby, T.J. and Stokes, C.R., 1984. Local Immune Responses of the Gut. CRC Press, Boca Raton, pp. 178-180.
Pierce, N.F. and Gowans, J.L., 1975. J. exp. Med., 142: 1550-1563.
Pointon, A.M., Byrt, D. and Heap, P., 1985. Aust. Vet. J., 62: 13.
Porter, P., 1979. Adoptive immunization of the neonate by breast factors. In immunology of Breast Milk. Eds. P.L. Ogra and D. Dayton. Raven Press, New York, pp 197-206.
Ross, R.F., Zimmerman-Erickson, B.J. and Young, T.F., 1985. Am. J. Vet. Res., 45: 1899.
Rowley, D., 1977. Aust. J. exp. Biol Med. Sci., 55: 1-18.
Saif, L.J., Redman, D.R., Smith, K.L. and Theil, K.W., 1983. Infection and Immunity, 41: 118-1131.
Sainte-Marie, G., 1962. J. Histochem. Cytochem., 10: 250-256.
Shek, P.N. and Sabiston, B.G., 1981. Immunology, 45: 349-356.
Sheldrake, R.F., Husband, A.J. and Watson, D.L., 1985. Res. vet. Sci., 38: 312-316.
Sheldrake, R.F., Gardner, I.A., Saunders, M.M. and Romalis, L.F., 1989. Aust. Vet. J. (Submitted).
Sheldrake, R.F., 1989a. Vet. Immunol. Immunopthol. (In Press).
Sheldrake, R.F., 1989b. Res. Vet. Sci. (In Press).
Sheldrake, R.F., Romalis, L.F. and Saunders, M.M., 1988. Res. Vet. Sci., 45: 369.
Webster, W.R., 1987. Antibiotics - use, misuse, resistance, growth promotion and non-antibiotic growth promotors. In Proceedings No. 95, Post Graduate Foundation, Faculty of Veterinary Science, University of Sydney, Sydney, Australia, p. 229.
Weng, C.-N., 1985 Serological Studies of Mycoplasma Infections in Pigs. Ph.D. Thesis, Cambridge, Cited in Aslib Index to Theses 35(1): 425.

## Claims

1. The use of a vegetable oil together with an antigenically active substance and an adjuvant in the manufacture of a vaccine composition for intraperitoneal administration to stimulate an IgA response in mucosal infections.

2. The use according to claim 1 wherein the mucosal infection is an enteric or respiratory infection.

3. The use according to claim 2 wherein the enteric infection is a bacterial infection.

4. The use according to any one of the preceding claims for the treatment of mucosal infections of livestock.

5. The use according to any one of the preceding claims wherein said vegetable oil comprises safflower oil or sunflower oil.

6. The use according to any one of the preceding claims wherein said adjuvant is selected from saponin, purified mycobacterial cell wall extracts (muramyldipeptide) or killed mycobacterium.

7. The use according to any one of the preceding claims wherein the composition is in the form of a stable vegetable oil-in-water emulsion and further comprises an emulsifier.

8. The use according to claim 7, wherein said emulsifier is phosphatidyl choline.

9. The use according to any of the preceding claims wherein the antigenically active substance comprises E.coli antigens.

10. The use according to any of claims 1 to 8 wherein the antigenically active substance comprises Mycoplasma hyopneumoniae antigens.

11. The use according to any of claims 1 to 8 wherein the antigenically active substance comprises S. typhimurim antigens.

12. The use according to claim 9 for stimulating a protective immune response against post-weaning enteritis in pigs.

13. The use according to claim 10 for stimulating a protective immune response against enzootic pneumonia in pigs.

14. The use according to claim 11 for stimulating a protective immune response against post-weaning enteritis in lambs.

15. A vaccine composition comprising an antigen selected from E.coli, M. hyopneumoniae and S. typhimurim and a vegetable oil vehicle selected from sunflower and safflower oil and an adjuvant, wherein said composition stimulates an IgA response in an animal following intraperitoneal administration.

## Patentansprüche

1. Verwendung eines Pflanzenöls zusammen mit einer antigenwirksamen Substanz und einem Adjuvans bei der Herstellung einer Impfstoffzusammensetzung für intraperitoneale Verabreichung zur Stimulation einer IgA-Reaktion bei Schleimhautinfektionen.

2. Verwendung nach Anspruch 1, worin die Schleimhautinfektion eine Darm- oder Atemwegsinfektion ist.

3. Verwendung nach Anspruch 2, worin die Darminfektion eine bakterielle Infektion ist.

4. Verwendung nach einem der vorherigen Ansprüche zur Behandlung von Schleimhautinfektionen bei Nutztieren.

5. Verwendung nach einem der vorherigen Ansprüche, worin das Pflanzenöl Safloröl oder Sonnenblumenöl umfaßt.

6. Verwendung nach einem der vorherigen Ansprüche, worin das Adjuvans ausgewählt ist aus Saponin, gereinigten Mycobakterienzellwandextrakten (Muramyldipeptid) oder abgetötetem Mycobakterium.

7. Verwendung nach einem der vorherigen Ansprüche, worin die Zusammensetzung in Form einer stabilen Pflanzenölin-Wasser-Emulsion vorliegt und ferner einen Emulgator enthält.

8. Verwendung nach Anspruch 7, worin der Emulgator Phosphatidylcholin ist.

9. Verwendung nach einem der vorherigen Ansprüche, worin die antigenwirksame Substanz E. coli-Antigene umfaßt.

10. Verwendung nach einem der Ansprüche 1 bis 8, worin die antigenwirksame Substanz Mycoplasma hyopneumoniae-Antigene umfaßt.

11. Verwendung nach einem der Ansprüche 1 bis 8, worin die antigenwirksame Substanz S. typhimurim-Antigene umfaßt.

12. Verwendung nach Anspruch 9 zur Stimulation einer schützenden Immunreaktion gegen Enteritis bei Schweinen nach der Entwöhnung.

13. Verwendung nach Anspruch 10 zur Stimulation einer schützenden Immunreaktion gegen enzootische Pneumonie bei Schweinen.

14. Verwendung nach Anspruch 11 zur Stimulation einer schützenden Immunreaktion gegen Enteritis bei Lämmern nach der Entwöhnung.

15. Impfstoffzusammensetzung umfassend ein Antigen ausgewählt aus E. coli, M. hyopneumoniae und S. typhimurim und einem Pflanzenölträger ausgewählt aus Sonnenblumen- und Safloröl und einem Adjuvans, worin die Zusammensetzung nach intraperitonealer Verabreichung in einem Tier eine IgA-Reaktion stimuliert.

## Revendications

1. Utilisation d'une huile végétale ainsi que d'une substance possédant une activité antigénique et d'un adjuvant dans la fabrication d'une composition vaccinale destinée à être administrée par voie intrapéritonéale pour stimuler une réponse à IgA en cas d'infection des muqueuses.

2. Utilisation selon la revendication 1 dans laquelle l'infection des muqueuses est une infection entérique ou respiratoire.

3. Utilisation selon la revendication 2 dans laquelle l'infection entérique est une infection bactérienne.

4. Utilisation selon l'une des revendications précédentes dans le traitement d'infections des muqueuses chez le bétail.

5. Utilisation selon l'une des revendications précédentes dans laquelle ladite huile végétale comprend l'huile de carthame ou l'huile de tournesol.

6. Utilisation selon l'une des revendications précédentes dans laquelle ledit adjuvant est choisi entre la saponine, des extraits purifiés de paroi cellulaire mycobactérienne (muramyldipeptide) ou des mycobactéries tuées.

7. Utilisation selon l'une des revendications précédentes dans laquelle la composition se trouve sous la forme d'une émulsion stable d'huile végétale dans de l'eau et comprend en outre un émulsifiant.

8. Utilisation selon la revendication 7 dans laquelle ledit émulsifiant est la phosphatidyl choline.

9. Utilisation selon l'une des revendications précédentes dans laquelle la substance possédant une activité antigénique comprend des antigènes d'E. coli.

10. Utilisation selon l'une des revendications 1 à 8 dans laquelle la substance possédant une activité antigénique comprend des antigènes de Mycoplasma hyopneumoniae.

11. Utilisation selon l'une des revendications 1 à 8 dans laquelle la substance possédant une activité antigénique comprend des antigènes de S. typhimurium.

12. Utilisation selon la revendication 9 pour stimuler une réponse immunitaire protectrice contre l'entérite consécutive au sevrage chez le porc.

13. Utilisation selon la revendication 10 pour stimuler une réponse immunitaire protectrice contre la pneumonie entozoaire chez le porc.

14. Utilisation selon la revendication 11 pour stimuler une réponse immunitaire protectrice contre l'entérite consécutive au sevrage chez le mouton.

15. Composition vaccinale comprenant un antigène choisi entre des antigènes d'E. coli, de Mycoplasma hyopneumoniae et de S. typhimurium et un véhicule à base d'huile végétale choisi entre l'huile de tournesol et l'huile de carthame et un adjuvant, ladite composition stimulant une réponde à IgA chez un animal après administration intrapéritonéale.
